# EUROPÄISCHE PATENTSCHRIFT

(11) **EP 1 648 355 B1**
(45) Veröffentlichungstag und Bekanntmachung des Hinweises auf die Patenterteilung: **23.07.2008**
(21) Anmeldenummer: 04737395.6
(22) Anmeldetag: 23.07.2004
(51) Int. Cl.: A61F 5/00

(54) **STEUERBARES MAGENBAND**
ADJUSTABLE STOMACH BAND
ANNEAU GASTRIQUE COMMANDE

(30) Priorität: 25.07.2003 AT 11802003; 24.02.2004 AT 2842004
(43) Veröffentlichungstag der Anmeldung: 26.04.2006
(73) Patentinhaber: Lechner, Wolfgang, 3441 Judenau/Pixendorf (AT)
(72) Erfinder: Lechner, Wolfgang, 3441 Judenau/Pixendorf (AT)
(74) Vertreter: Sonn & Partner Patentanwälte
(86) Internationale Anmeldenummer: PCT/AT2004/000265
(87) Internationale Veröffentlichungsnummer: WO 2005/009305

(56) Entgegenhaltungen:
- EP-A- 0 876 808
- WO-A-20/04014245
- US-A- 4 592 339
- US-B1- 6 460 543
- US-B1- 6 475 136

## Beschreibung

Die Erfindung betrifft ein steuerbares Magenband mit einem nicht dehnbaren Rücken und einer stomaseitig vom Rücken angeordneten Kammer zur Steuerung der Stomaeinengung durch Zu- bzw. Abfuhr von Flüssigkeit in die bzw. aus der Kammer, wobei eine zweite Kammer vorgesehen ist.

Die Erfindung bezieht sich auf eine Weiterentwicklung des steuerbaren Magenbandes, wie es von mehreren Firmen in prinzipiell gleicher Bauform angeboten wird (z.B. Schwedenband der Fa.Obtech (Johnson & Johnson), Lapband der Fa.Bioenterics,..) Es handelt sich hierbei um ein zur Restriktion der Nahrungsaufnahme eingesetztes Band, das um den obersten Magenteil bzw. Ösophagus herumgeschlungen und verschlossen wird.

Die WO 01/24742 A1 beschreibt ein Magenband, welches gürtelförmig um den Magen herumgelegt und befestigt wird. Eine Einstellung der Einengung des Stomas ist rein mechanisch durch Einengung des Bandes möglich.

Die US 4 592 339 A beschreibt ein Magenband, bei dem an der dem Magen zugewandten Seite des Bandes eine Kammer angeordnet ist, die mit Flüssigkeit aufgefüllt werden kann. Dadurch ist die Steuerung der Stomaweite möglich. Über einen subkutan eingenähten Port, der über einen Schlauch mit der Kammer des Magenbandes verbunden ist, kann eine Flüssigkeitsfüllung und Entleerung des Systems durchgeführt werden.

Schließlich zeigt die WO 03/020183 A1 ein Magenband, welches zur Schonung des Magens mit einem viskoelastischen Material umgeben ist.

Die US 6 460 543 B1 beschreibt ein Magenband der gegenständlichen Art, bei welchem eine Steuerung der Stomaeinengung durch Flüssigkeitsverschiebung beispielsweise über eine Fernsteuerung vorgenommen werden kann. Eine Autoregulation der Stomaeinengung in Abhängigkeit der Nahrungsaufnahme ist nicht vorgesehen.

Die derzeit verwendeten Magenbänder bringen in der Mehrzahl der Fälle sehr gute Langzeitergebnisse hinsichtlich Gewichtsreduktion und Patientenzufriedenheit. Dennoch gibt es einige Probleme die besonders bei hoher Bandauffüllung in den Vordergrund treten. Viele Patienten berichten dann über die unangenehme Erscheinung des Speichel-Erbrechens bzw. Herauswürgens, v.a. beim flachen Liegen. Speisereste können lange oberhalb des Stomas in der Speiseröhre verbleiben, hier zu gären beginnen und dadurch neben einem unangenehmen Mundgeruch eine Schleimhautreizung mit entsprechenden Schmerzen hervorrufen. Die ununterbrochen bestehende hohe Engstellung des Stomas führt wie bei einer Achalasie im Verlauf von Monaten zu einer zunehmenden Ausdehnung der Speiseröhre, wodurch schließlich die Ösophagus-Sensibilität schwindet und die Bandwirkung verloren geht, was dann zu einer Gewichtszunahme trotz liegendem hoch aufgefülltem Magenband führt.

Das Problem bei derzeit verwendeten Magenbändern besteht darin, dass die gewählte Auffüllung und damit Stomaweite ständig gleich bleibt, obwohl nur eine Restriktion der Nahrungsaufnahme angestrebt wird.

Ausgehend von dem Stand der Technik beim derzeit verwendeten Magenband zielt die Erfindung darauf ab, ein Magenband zu schaffen, bei dem eine vorzugsweise automatische Einstellung der Stomaweite möglich ist. Anzustreben wäre ein Magenband, das nur bei der Nahrungsaufnahme eng gestellt ist, die übrige Zeit aber ausreichend weit ist, um keine negativen Folgewirkungen zu erzeugen. Das Band soll sich beim Essen einengen und nach Abschluss der Nahrungsaufnahme wieder weit werden.

Gelöst wird die erfindungsgemäße Aufgabe einerseits dadurch, dass die zweite Kammer stomaseitig vom Rücken vorgesehen ist, und mit der ersten Kammer in Verbindung steht, so dass die Steuerung der Stomaeinengung durch Verschiebung der Flüssigkeit zwischen der einen Kammer und der anderen Kammer erreicht wird. Somit ist eine Regulation des Magenbandes und somit der Stomaeinengung durch Verschiebung der Flüssigkeit zwischen zwei Kammern ohne das Hinzufügen oder Ableiten der Flüssigkeit von bzw. nach außen möglich. Die Ungleichverteilung der Flüssigkeit zwischen den beiden Kammern bewirkt ein engeres Stoma in Höhe der höher aufgefüllten Kammer. Dadurch wird ein Magenband geschaffen, welches einen Flüssigkeitskreislauf aufweist.

Gelöst wird die erfindungsgemäße Aufgabe auch dadurch, dass die zweite Kammer stomaseitig vom Rücken vorgesehen ist, und als Sensor zur Erfassung eines Druckanstiegs im Magen bzw. Ösophagus ausgebildet ist, und dass die andere Kammer mit einem Reservoir verbunden ist, so dass in Abhängigkeit des über die sensorische Kammer erfassten Drucks die Steuerung der Stomaeinengung durch Verschiebung der Flüssigkeit zwischen dem Reservoir und der Stoma-einengenden Kammer erreicht wird. Durch diese Ausführungsform wird ein Zwei-Kreislauf-System gebildet, wobei die sensorische Kammer einen Flüssigkeitsraum darstellt und die Stoma-einengende Kammer zusammen mit dem Reservoir den zweiten Flüssigkeitsraum darstellt. Somit kommt es nicht, wie beim Ein-Kammerkreislauf-System zu einer Flüssigkeitsverschiebung von der einen Kammer zur anderen Kammer, sondern in Abhängigkeit des über die sensorische Kammer erfassten Drucks zu einer Verschiebung der Flüssigkeit vom Reservoir zur Stoma-einengenden Kammer und umgekehrt. Die beiden Kammern stehen somit in Wirkverbindung miteinander.

Die Kammern sind vorzugsweise nebeneinander angeordnet, wobei die Stoma-einengende Kammer aboral gelegen ist.

Die zweite Kammer kann auch in zwei kommunizierende Kammern unterteilt sein, welche die Stoma-einengende Kammer an beiden Seiten begrenzen. Dadurch wird die Stabilität des Magenbandes erhöht.

Ebenso ist es möglich, dass die Kammern in Bezug auf den Magen bzw. Ösophagus übereinander angeordnet sind, wobei die sensorische Kammer an der Magenwand angeordnet ist.

Dabei kann zwischen den Kammern eine Schicht angeordnet sein.

Zum Verschieben der Flüssigkeit von einer Kammer bzw. dem Reservoir in die Stoma-einengende Kammer und umgekehrt ist vorzugsweise eine Einrichtung zum Pumpen der Flüssigkeit vorgesehen.

Dabei ist die Pumpeinrichtung entweder durch eine elektrische oder eine mechanische angetriebene Pumpe, beispielsweise eine Saugdruckpumpe gebildet. Im Fall der Verwendung einer mechanisch angetriebenen Pumpe, beispielsweise einer Saugdruckpumpe wird jene Energie, welche von der sensorischen Kammer aufgenommen wird zum Aufpumpen der Stoma-einengenden Kammer verwendet.

Zur Erzielung einer Autoregulation der Stomaeinengung durch das Magenband ist vorteilhafterweise eine Einrichtung zur Erfassung der Essensaktivität vorgesehen.

Dabei kann die Essensaktivität durch Erfassung der Schluckaktivität, Erfassung des Druckes an der Magenwand bzw. Ösophaguswand oder Erfassung der peristaltischen Welle geschehen. Zur Erfassung des Druckes an der Magenwand bzw. Ösophaguswand kann in der sensorischen Kammer ein Drucksensor vorgesehen sein, welcher mit einer elektronischen Schaltung verbunden ist.

Gemäß einem weiteren Merkmal der Erfindung ist vorgesehen, dass die Erfassungseinrichtung mit der Pumpeinrichtung verbunden ist, so dass nach Erfassung der Essensaktivität Flüssigkeit von der sensorischen Kammer bzw. dem Reservoir in die Stoma-einengende Kammer gepumpt wird und eine bestimmte Zeit nach Erfassung der Beendigung der Essensaktivität die Flüssigkeit wieder von der Stoma-einengenden Kammer in die zweite Kammer bzw. das Reservoir zurückbewegt wird. Dadurch kann eine automatische Stomaeinengung in Abhängigkeit der Nahrungsaufnahme stattfinden.

Gemäß einer Ausführungsvariante eines Magenbandes mit nur einem Flüssigkeitskreislauf auf rein mechanischem Wege sind die Kammern über eine Hilfskammer mit Windkesselfunktion miteinander verbunden, wobei zwischen der zweiten Kammer und der Hilfskammer ein Ventil angeordnet ist, welches einen Flüssigkeitstransport nur von der zweiten Kammer zur Hilfskammer zulässt, und zwischen der Hilfskammer und der Stoma-einengenden Kammer ein weiteres Ventil angeordnet ist, welches einen Flüssigkeitstransport nur von der Hilfskammer zur Stoma-einengenden Kammer zulässt.

Im Fall eines Magenbandes mit zwei Flüssigkeitskreisläufen kann in der Verbindung zwischen dem Reservoir und der Stoma-einengenden Kammer ebenfalls eine Hilfskammer mit Windkesselfunktion angeordnet sein. Dadurch kann die durch den Druckanstieg im Magen bzw. Ösophagus auftretende Energie kurzzeitig zwischengespeichert und erst dann zum Aufpumpen der Stoma-einengenden Kammer verwendet werden. Dadurch wir erreicht, dass erst nach Abklingen der peristaltischen Welle die Stoma-einengende Kammer aktiviert wird.

Um einen Flüssigkeitsaustausch zwischen der Stoma-einengenden Kammer und der zweiten Kammer bzw. dem Reservoir zu gewährleisten, ist eine Einrichtung zur Durchführung eines Flüssigkeitsaustauschs von der Stoma-einengenden Kammer zur zweiten Kammer bzw. zum Reservoir vorgesehen.

Diese Einrichtung zur Durchführung des Flüssigkeitsaustausches von der Stoma-einengenden Kammer zur zweiten Kammer bzw. zum Reservoir kann durch eine gemeinsame Trennwand zwischen den Kammern bzw. der Kammer und dem Reservoir mit darin enthaltenen Mikroporen gebildet sein. Über diese Mikroporen in der Trennwand zwischen den aneinander angrenzenden Kammern des Magenbandes bzw. der Kammer und dem Reservoir findet ein langsamer kontinuierlicher Flüssigkeitsaustausch statt.

Die Einrichtung zur Durchführung eines Flüssigkeitsaustauschs kann auch durch einen Rückflusskanal zwischen den Kammern bzw. der Stoma-einengenden Kammer und dem Reservoir gebildet sein.

Darin kann ein Drosselventil oder dgl. angeordnet sein, welches den Flüssigkeitsstrom drosselt und nur in eine Richtung zuläßt.

Gemäß einem weiteren Merkmal der Erfindung ist es vorgesehen, dass die sensorische Kammer mit einem Magenschrittmacher oder einem elektrische Impulse abgebenden Gerät in Verbindung steht, so dass in Abhängigkeit des in der sensorischen Kammer erfassten Drucks im Magen bzw. Ösophagus eine Stimulierung der Magenwand durch elektrische Impulse erzielbar ist. Dadurch kann während der Nahrungsaufnahme eine Stimulierung des Magens oder des Ösophagus durchgeführt werden.

Zusätzlich kann eine weitere flüssigkeitsgefüllte Kammer zur Anpassung des Magenbandes vorgesehen sein, welche weder eine sensorische noch eine Stoma-einengende Funktion im Rahmen des steuerbaren Magenbandes aufweist, sondern zur Anpassung des Magenbandes an die jeweiligen Gegebenheiten dient. Diese weitere Kammer wird über der sensorischen und Stoma-einengenden Kammer des Magenbandes angeordnet und zusammen um den obersten Magenteil bzw. Ösophagus herumgeschlungen und befestigt.

Vorteilhafterweise ist die weitere Kammer mit einem subkutan anzuordnenden Port verbunden, so dass durch Zuführung bzw. Abführung von Flüssigkeit über den Port die Kammer mit Flüssigkeit befüllt bzw. der Kammer Flüssigkeit entzogen werden kann und so eine Anpassung des Magenbandes erfolgen kann. Natürlich können auch mehrere solche Kammern zur Anpassung des Magenbandes vorgesehen sein.

Die Erfindung wird anhand der beigefügten Abbildungen näher erläutert.

Darin zeigen:
Fig. 1 einen schematischen Querschnitt durch eine Ausführungsform eines Magenbandes mit einem Flüssigkeitskreislauf im Zustand der Gleichverteilung der Flüssigkeitsfüllung in den beiden Kammern;
Fig.2 einen schematischen Querschnitt durch das Magenband gemäß Fig. 1 nach Flüssigkeitsverlagerung in die Stoma-einengende Kammer;
Fig. 3 einen schematischen Querschnitt durch eine weitere Ausführungsform eines steuerbaren Magenbandes mit zwei Flüssigkeitskreisläufen mit elektrisch angetriebener Pumpe;
Fig. 4 einen schematischen Querschnitt durch eine weitere, mechanische Ausführungsform eines steuerbaren Magenbandes mit zwei Flüssigkeitskreisläufen;
Fig. 5 eine perspektivische Ansicht einer weiteren Ausführungsform eines steuerbaren Magenbandes; und
Fig. 6a und 6b Schnittbilder durch das Magenband gemäß Fig. 5 bei unterschiedlicher Füllung der Stoma-einengenden Kammer.

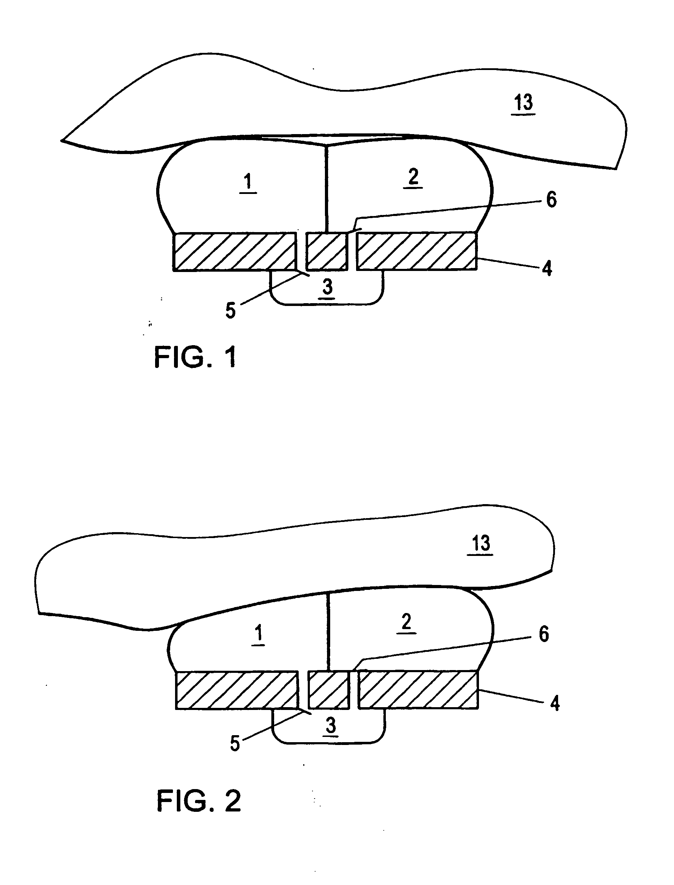

Das in den Fig. 1 und 2 dargestellte Magenband hat wie die derzeit verwendeten Bänder einen nicht dehnbaren Rücken 4. An der der Magenwand 13 zugewandten Seite des Rückens 4 befinden sich zwei Kammern 1, 2, welche miteinander verbunden sind, so dass eine Verschiebung der Flüssigkeit zwischen den Kammern 1 und 2 möglich ist. Die der Magenwand 13 zugewandten Kammern 1, 2 verlaufen zirkulär um den Magen bzw. den Ösophagus wie bei herkömmlichen Magenbändern mit nur einer Kammer. Beim dargestellten Ausführungsbeispiel befindet sich an der der Magenwand 13 abgewandten Außenseite des Rückens 4 eine Hilfskammer 3 mit Windkesselfunktion. Die Hilfskammer 3 ist mit beiden Kammern 1, 2 verbunden. Ein Ventil 5 zwischen der Kammer 1 und der Hilfskammer 3 gestattet den Flüssigkeitsübertritt von der Kammer 1 in die Hilfskammer 3. Ein weiteres Ventil 6 zwischen der Stoma-einengenden Kammer 2 und der Hilfskammer 3 gestattet einen Flüssigkeitsübertritt von der Hilfskammer 3 in die Stoma-einengende Kammer 2. Beim Durchtritt fester Speisen durch das Stoma erhöht sich somit der Druck in den beiden an die Magenwand 13 angrenzenden Kammern 1, 2 von einem Ruhedruck p1 auf einen Druck p2. Flüssigkeit wird somit von der Kammer 1 in die Hilfskammer 3 gepresst. Das Ventil 5 verhindert einen Rückstrom von der Hilfskammer 3 in die Kammer 1. Auf Grund der Windkesselfunktion speichert die Hilfskammer 3 den erhöhten Druck p2. Nach Durchtritt des Bissens durch das Stoma sinkt der Flüssigkeitsdruck in den beiden Magenwand-nahen Kammern 1 und 2 wieder auf den Ruhedruck p1 ab. Die unter dem erhöhten Druck p2 stehende Hilfskammer 3 entleert die Flüssigkeit danach in die Stoma-einengende Kammer 2. Somit wird über den hier rein mechanischen Pumpmechanismus bei der Passage von festen Nahrungsmitteln durch das Stoma Flüssigkeit aus der Kammer 1 in die Stoma-einengende Kammer 2 verschoben. Dadurch wird die Stomaöffnung in Höhe der Kammer 2 enger gestellt, wie in Fig. 2 schematisch dargestellt. Die Flüssigkeitsverschiebung erfolgt während des Essens kontinuierlich. Nach dem Essen wird das Band nach einer bestimmten Zeit wieder weiter gestellt, indem ein Rückfluss von der Stoma-einengenden Kammer 2 in die Kammer 1 eingeleitet wird. Der optimale Zeitpunkt für diesen Flüssigkeitsaustausch muss in klinischen Studien festgestellt werden. Beim Ausführungsbeispiel gemäß den Figuren 1 und 2 handelt es sich um ein Magenband mit durch einen durch die Kammern 1, 2 und die Hilfskammer 3 gebildeten Flüssigkeitskreislauf. Beim dargestellten Beispiel erfolgt der Flüssigkeitsaustausch über Mikroporen, welche in der Scheidewand zwischen den aneinander angrenzenden Kammern 1 und 2 angeordnet sind. Dadurch findet ein langsamer kontinuierlicher Flüssigkeitsausgleich statt. Die Steuerung der Pumpvorgänge kann elektronisch oder mechanisch durchgeführt werden.

Das Ziel der autoregulatorischen Veränderung der Stomaweite wird somit durch eine Flüssigkeitsverschiebung von einer Kammer in die andere Kammer erreicht. Gegenüber dem Ausgangszustand der in beiden Kammern gleichverteilten Flüssigkeitsmenge bedeutet eine Ungleichverteilung eine Stomaeinengung im Bereich der einen und eine Stomaerweitung in Höhe der anderen Kammer. Im Effekt für den Magenbandträger bedeutet es eine Stomaeinengung und damit Erschwerung des Essvorganges.

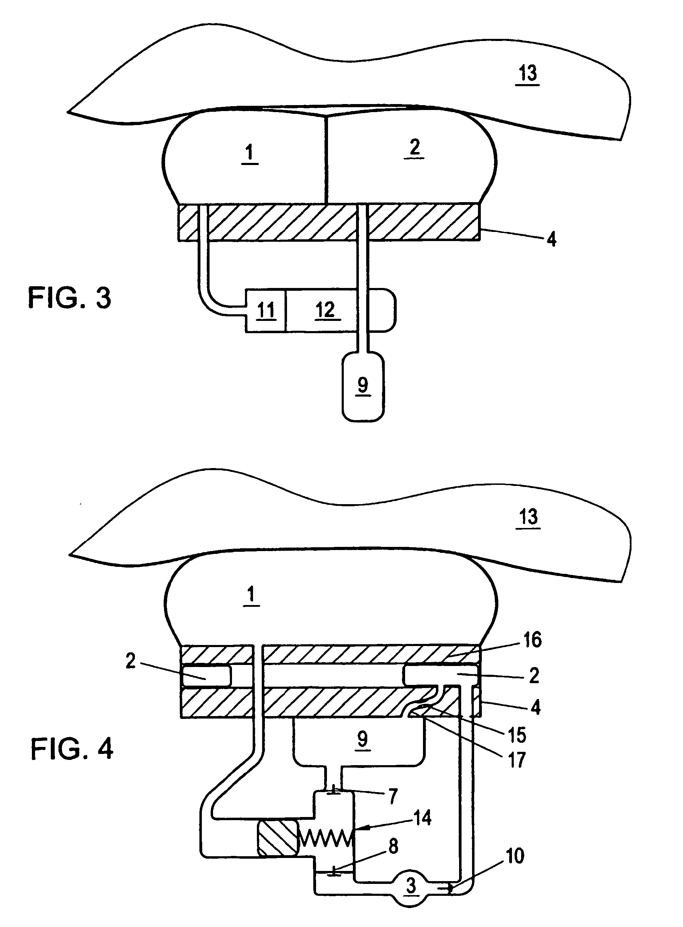

Die in den Fig. 3 und 4 dargestellten Ausführungsformen eines Magenbandes zeigen Varianten, für welchen zwei Flüssigkeitskreisläufe vorgesehen sind. Dabei hat das Magenband wie die derzeit verwendeten Bänder einen nicht dehnbaren Rücken 4. An jener Seite des Rückens 4, welcher der Magenwand 13 zugewandt ist, befinden sich zwei Kammern 1, 2, welche miteinander in Wirkverbindung stehen. Die Kammer 1, welche im Bezug auf den Magen kranial angeordnet ist, hat die Funktion eines Sensors und erfasst die während der Nahrungsaufnahme hervorgerufenen Bewegungen des Magens bzw. Ösophagus. Die neben der sensorischen Kammer 1 gelegene Kammer 2 dient zur Stomaeinengung und steht mit einem Reservoir 9 in Verbindung. Aus diesem Reservoir 9 kann Flüssigkeit in die Kammer 2 gepumpt und wieder von dieser in das Reservoir 9 zurück befördert werden. Zur Wirkverbindung zwischen der Kammer 1 und 2 dient beispielsweise eine elektronische Schaltung 11, welche den von der sensorischen Kammer 1 aufgenommenen Druck detektiert und eine elektrische Pumpe 12 entsprechend ansteuert. Die elektrische Pumpe 12 befördert Flüssigkeit aus dem Reservoir 9 in die Kammer 2 und bewirkt somit eine Einengung des Stoma. Diese Einengung des Stoma erfolgt somit nach Feststellung der Nahrungspassage durch die Kammer 1. Der Druck im Inneren der Kammer 1 steigt beim Durchtritt von geschluckten Speisen an, so dass ein steuerbares Magenband herkömmlicher Bauart als Sensor für Schluckaktivität verwendet werden kann. Durch die Verschiebung von Flüssigkeit aus dem Reservoir 9 in die Kammer 2 wird die Einengung des Stomas bewirkt, durch Rückverlagerung der Flüssigkeit in das Reservoir 9 weitet sich das Stoma.

Fig. 4 zeigt eine Variante des erfindungsgemäßen steuerbaren Magenbandes bei dem die Verbindung zwischen der Kammer 1 und der mit dem Reservoir 9 verbundenen Kammer 2 über eine Saugdruckpumpe 14 erfolgt. In diesem Fall wird der in der sensorischen Kammer 1 während der Nahrungsaufnahme ansteigende Druck zum Aufpumpen der Kammer 2 verwendet. Die Flüssigkeit in der sensorischen Kammer 1 betätigt somit die Saugdruckpumpe 14 und bewirkt durch entsprechend angeordnete Ventile 7, 8 einen Flüssigkeitsstrom vomReservoir 9 in die Kammer 2 und bewirkt somit ein Aufpumpen der Kammer 2 und in der Folge eine Einengung des Stoma. Durch die Anordnung einer Hilfskammer 3 mit Windkesselfunktion zwischen dem Ventil 8 und einem weiteren Ventil 10, kann ein verzögerter Flüssigkeitsstrom erfolgen und somit eine verzögerte Verengung des Stoma erzielt werden. Der Rückstrom der Flüssigkeit von der Kammer 2 in das Reservoir 9 kann über einen Rückflusskanal 17 erfolgen, in dem beispielsweise ein Drosselventil 15 angeordnet sein kann, welches eine langsame Auflösung der Stomaeinengung bewirkt. Somit wird nach Beendigung des Essens der Pumpmechanismus deaktiviert und ein langsamer Flüssigkeitsrückstrom aus der Kammer 2 in das Reservoir 9 erzielt. Der Flüssigkeitsrückstrom erfolgt ohne Energieaufwand auf Grund eines Druckgradienten zwischen der Stoma-einengenden Kammer 2 und dem Reservoir 9. Zwischen der Kammer 2 und dem Reservoir 9 besteht notwendig ein Druckgradient, da das Reservoir 9 außerhalb des Rückens 4 liegt und daher nicht die mit Auffüllung der Stoma-einengenden Kammer 2 einhergehende Erhöhung des Ruhedrucks mitmacht.

Die Ausführungsvariante gemäß Fig. 4 unterscheidet sich weiters darin von jener in Fig. 3, dass die beiden Kammern 1, 2 in Bezug auf die Magenwand 13 übereinander angeordnet sind. Dabei sind die Kammern 1, 2 radial übereinander angeordnet, wobei die sensorische Kammer 1 an der Magenwand 13 bzw. der Ösophaguswand angeordnet ist und die Kammer 2 über die Kammer 1 allenfalls unter Zwischenlage einer Schicht 16 angeordnet ist. Durch die Anordnung der Kammern 1, 2 übereinander ergibt sich eine andere Drucksituation als bei den nebeneinander angeordneten Kammern 1, 2. Im Falle der übereinander angeordneten Kammern 1, 2 würde es beim Ein-Kreislauf-System gemäß den Fig. 1 und 2 zu keinem Flüssigkeitsrückstrom von der Stoma-einengenden Kammer 2 in die sensorische Kammer 1 kommen, da in beiden Kammern 1, 2 derselbe Druck herrscht, da sich beide Kammern 1, 2 übereinander zwischen Magenwand 13 und Rücken 4 befinden. Eine Restriktion bei Flüssigkeitsverlagerung von der sensorischen Kammer 1 in die Stoma-einengende Kammer 2 findet nur dann statt, wenn die Stoma-einengende Kammer 2 schmäler ist als die sensorische Kammer 1. Damit ist zugleich auch das Problem des Druckgradienten bewältigt. Für die Kraft, welche die beiden Kammern 1, 2 auf die gemeinsame Grenzfläche A ausüben, gilt F = p . A. Auf Grund der größeren Fläche, mit der die sensorische Kammer 1 auf die gemeinsame Grenzfläche trifft, ist die Kraft der sensorischen Kammer 1 größer als die Kraft der Stoma-einengenden Kammer 2 mit der kleineren Kontaktfläche. Daher hat die Stoma-einengende Kammer 2 die Tendenz, sich in die sensorische Kammer 1 zu entleeren. Die Flüssigkeit strömt nach Beendigung des Essens in die sensorische Kammer 1 zurück und die Stomaweite nimmt wieder zu.

Die Variante mit der Saugdruckpumpe 14 gemäß Fig. 4 kommt ohne elektrische Energie aus. Die Kammern 1, 2 umfassen beispielsweise den gesamten Umfang.

Im Gegensatz zum Ein-Kreislauf-System gemäß den Fig. 1 und 2 gibt es beim Zwei-Kreislauf-System gemäß den Figuren 3 und 4 bei keiner Anordnung der Kammern 1, 2 Probleme mit dem Funktionieren der Restriktion. Es wird immer Flüssigkeit aus einem Reservoir 9 in die Stoma-einengende Kammer 2 verlagert und somit die Stomaweite notwendig verringert. Beim Ein-Kreislauf-System hingegen bewirkt eine Flüssigkeitsverlagerung von der sensorischen Kammer 1 in die Stoma-einengende Kammer 2 nur dann eine Stomaeinengung, wenn die Kammern 1, 2 nebeneinander angeordnet sind. Liegen die beiden Kammern 1, 2 radial übereinander, dann würde eine Flüssigkeitsverlagerung aus der einen Kammer 1 in die Stoma-einengende Kammer 2 zu keiner Veränderung der Stomaweite führen. Diese erreicht man nur dann, wenn jene Kammer, in welche die Flüssigkeit eingepumpt wird, schmäler ausgeführt ist, als jene Kammer aus der die Flüssigkeit entnommen wird. Ist die Stoma-einengende Kammer schmäler, dann ist sie bei gleicher Flüssigkeitsmenge höher und damit die Stomaweite enger. Die Verlagerung der Flüssigkeit aus der breiten Kammer in die schmale Kammer führt somit zu einer Stomaeinengung.

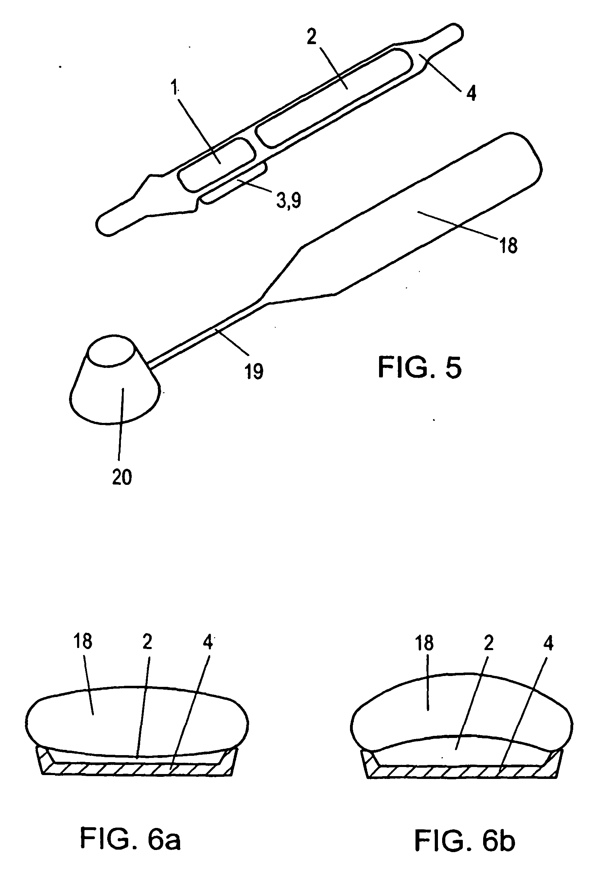

Fig. 5 zeigt eine perspektivische Ansicht einer weiteren Ausführungsform eines Magenbandes, wobei die sensorische Kammer 1 und die Stoma-einengende Kammer 2 nebeneinander entlang dem Umfang des Magenbandes auf dem nicht dehnbaren Rücken 4 angeordnet sind. Die Hilfskammer 3, über welche die Flüssigkeit von der Kammer 1 zur Stoma-einengenden Kammer 2 gepumpt wird bzw. ein Reservoir 9, welches in Verbindung mit der Stoma-einengenden Kammer 2 steht, befindet sich außerhalb des Rückens 4. Das Magenband wird um den obersten Magenteil bzw. Ösophagus herumgeschlungen und verschlossen. Im dargestellten Ausführungsbeispiel nehmen die beiden Kammer 1, 2 jeweils nur einen Teil der Circumferenz ein. Zusätzlich kann eine weitere Kammer 18 vorgesehen sein, welche weder eine sensorische noch eine Stoma-einengende Funktion im Rahmen des steuerbaren Magenbandes aufweist, sondern zur Anpassung des Magenbandes an die jeweiligen Gegebenheiten dient. Zu diesem Zweck wird die länglich ausgebildete Kammer 18 über den Kammern 1, 2 des Magenbandes angeordnet und zusammen um den obersten Magenteil bzw. Ösophagus herumgeschlungen und befestigt. Die Kammer 18 ist über eine entsprechende Leitung 19 in an sich bekannter Weise mit einem Port 20 verbunden, der subkutan angeordnet wird. Die Anpassung des Magenbandes kann somit über Zuführung bzw. Abführung von Flüssigkeit über den Port 20 in bzw. aus der Kammer 18 erfolgen.

Fig. 6a und 6b zeigt einen Schnitt durch ein derartiges Magenband gemäß Fig. 5 im Bereich der Stoma-einengenden Kammer 2. In der Fig. 6a ist die Stoma-einengende Kammer 2 wenig befüllt, so dass die Kammer 18 weniger an die Magenwand 13 (nicht dargestellt) gepresst wird als in der Situation gemäß Fig. 6b, bei der die Stoma-einengende Kammer 2 mit entsprechender Flüssigkeit befüllt wird und somit die Kammer 18 gegen die Magenband 13 bzw. Ösophaguswand gepresst wird und es zu einer Einengung des Stoma kommt. Durch diese zusätzliche an der Autoregulation nicht teilnehmenden Kammer 18 kann somit eine optimale Adaptierung des Magenbands von außen über den Port 20 erfolgen.

Die sensorische Funktion des Magenbandes ermöglicht einen neuen Zugang zum Konzept eines Magenschrittmachers. Durch die Kombination eines als Sensor arbeitenden Magenbandes mit einem Schrittmacher kann die Stimulierung der Magenwand durch elektrische Impulse auf die Zeiten der Nahrungsaufnahme beschränkt werden. Auch wäre es möglich durch entsprechende Platzierung der Schrittmachersonden im Bereich des unteren Ösophagussphinkter eine Aktivierung dieses Schließmuskels zu bewirken und damit eine Restriktion auf die Nahrungspassage. Die Restriktion bewirkt dann statt der aktiven Kammer eine physiologisch gegebene Struktur.

## Patentansprüche

1. Steuerbares Magenband mit einem nicht dehnbaren Rücken (4) und einer stomaseitig vom Rücken (4) angeordneten Kammer (2) zur Steuerung der Stomaeinengung durch Zu- bzw. Abfuhr von Flüssigkeit in die bzw. aus der Kammer (2), **dadurch gekennzeichnet, dass** eine zweite Kammer (1) stomaseitig vom Rücken (4) vorgesehen ist, welche mit der ersten Kammer (2) in Verbindung steht, so dass die Steuerung der Stomaeinengung durch Verschiebung der Flüssigkeit zwischen der einen Kammer (1) und der anderen Kammer (2) erreicht wird.

2. Steuerbares Magenband mit einem nicht dehnbaren Rücken (4) und einer stomaseitig vom Rücken (4) angeordneten Kammer (2) zur Steuerung der Stomaeinengung durch Zu- bzw. Abfuhr von Flüssigkeit in die bzw. aus der Kammer (2), **dadurch gekennzeichnet, dass** eine zweite Kammer (1) stomaseitig vom Rücken (4) vorgesehen ist, welche als Sensor zur Erfassung eines Druckanstiegs im Magen bzw. Ösophagus ausgebildet ist, und dass die andere Kammer (2) über die sensorische Kammer (1) mit einem Reservoir (9) verbunden ist, so dass in Abhängigkeit des erfassten Drucks die Steuerung der Stomaeinengung durch Verschiebung der Flüssigkeit zwischen dem Reservoir (9) und der Stoma-einengenden Kammer (2) erreicht wird.

3. Magenband nach Anspruch 1 oder 2, **dadurch gekennzeichnet, dass** die Kammern (1, 2) nebeneinander angeordnet sind, wobei die Stoma-einengende Kammer (2) aboral gelegen ist.

4. Magenband nach Anspruch 3, **dadurch gekennzeichnet, dass** die zweite Kammer (1) in zwei kommunizierende Kammern unterteilt ist, welche die Stoma-einengende Kammer (2) an beiden Seiten begrenzen.

5. Magenband nach Anspruch 1 oder 2, **dadurch gekennzeichnet, dass** die Stoma-einengende Kammer (2) und die sensorische Kammer (1) im Bezug auf den Magen bzw. Ösophagus übereinander angeordnet sind, wobei die sensorische Kammer (1) an der Magenwand (13) angeordnet ist.

6. Magenband nach Anspruch 5, **dadurch gekennzeichnet, dass** zwischen den Kammern (1, 2) eine Schicht (16) angeordnet ist.

7. Magenband nach einem der Ansprüche 1 bis 6, **dadurch gekennzeichnet, dass** eine Einrichtung zum Pumpen der Flüssigkeit von einer Kammer (1) bzw. dem Reservoir (9) in die Stoma-einengende Kammer (2) und umgekehrt vorgesehen ist.

8. Magenband nach Anspruch 7, **dadurch gekennzeichnet, dass** die Pumpeinrichtung durch eine elektrische Pumpe (12) gebildet ist.

9. Magenband nach Anspruch 7, **dadurch gekennzeichnet, dass** die Pumpeinrichtung durch eine mechanisch angetriebene Pumpe, beispielsweise eine Saugdruckpumpe (14) gebildet ist.

10. Magenband nach einem der Ansprüche 1 bis 9, **dadurch gekennzeichnet, dass** eine Einrichtung zur Erfassung der Essensaktivität vorgesehen ist.

11. Magenband nach Anspruch 10, **dadurch gekennzeichnet, dass** die Erfassungseinrichtung durch eine Einrichtung zur Erfassung der Schluckaktivität gebildet ist.

12. Magenband nach Anspruch 10, **dadurch gekennzeichnet, dass** die Erfassungseinrichtung durch eine Einrichtung zur Erfassung des Druckes an der Magenwand (13) bzw. Ösophaguswand gebildet ist.

13. Magenband nach Anspruch 12, **dadurch gekennzeichnet, dass** in der sensorischen Kammer (1) ein Drucksensor zur Erfassung des Druckes an der Magenwand (13) bzw. Ösophaguswand vorgesehen ist, welcher mit einer elektronischen Schaltung (11) verbunden ist.

14. Magenband nach Anspruch 10, **dadurch gekennzeichnet, dass** die Erfassungseinrichtung durch eine Einrichtung zur Erfassung der peristaltischen Welle gebildet ist.

15. Magenband nach einem der Ansprüche 10 bis 14, **dadurch gekennzeichnet, dass** die Erfassungseinrichtung mit der Pumpeinrichtung verbunden ist, so dass nach Erfassung der Essensaktivität Flüssigkeit von der sensorischen Kammer (1) bzw. dem Reservoir (9) in die Stoma-einengende Kammer (2) gepumpt wird und eine bestimmte Zeit nach Erfassung der Beendigung der Essensaktivität die Flüssigkeit wieder von der Stoma-einengenden Kammer (2) in die zweite Kammer (1) bzw. das Reservoir (9) zurückbewegt wird.

16. Magenband nach Anspruch 1, **dadurch gekennzeichnet, dass** die Kammern (1, 2) über eine Hilfskammer (3) miteinander verbunden sind, wobei zwischen der zweiten Kammer (1) und der Hilfskammer (3) ein Ventil (5) angeordnet ist, welches einen Flüssigkeitstransport nur von der zweiten Kammer (1) zur Hilfskammer (3) zulässt, und dass zwischen der Hilfskammer (3) und der Stoma-einengenden Kammer (2) ein weiteres Ventil (6) angeordnet ist, welches einen Flüssigkeitstransport nur von der Hilfskammer (3) zur Stoma-einengenden Kammer (2) zulässt.

17. Magenband nach einem der Ansprüche 2 bis 15, **dadurch gekennzeichnet, dass** zwischen dem Reservoir (9) und der Stoma-einengenden Kammer (2) eine Hilfskammer (3) mit Windkesselfunktion angeordnet ist.

18. Magenband nach einem der Ansprüche 1 bis 17, **dadurch gekennzeichnet, dass** zwischen der Stoma-einengenden Kammer (2) und der zweiten Kammer (1) bzw. dem Reservoir (9) eine Einrichtung zur Durchführung eines Flüssigkeitsaustauschs von der Stoma-einengenden Kammer (2) zur zweiten Kammer (1) bzw. zum Reservoir (9) vorgesehen ist.

19. Magenband nach Anspruch 18, **dadurch gekennzeichnet, dass** die Einrichtung zur Durchführung eines Flüssigkeitsaustauschs durch eine gemeinsame Trennwand zwischen den Kammern (1, 2) bzw. der Stoma-einengenden Kammer (2) und dem Reservoir (9) mit darin enthaltenen Mikroporen gebildet ist.

20. Magenband nach Anspruch 18, **dadurch gekennzeichnet, dass** die Einrichtung zur Durchführung eines Flüssigkeitsaustauschs durch einen zwischen den Kammern (1, 2) bzw. der Stoma-einengenden Kammer (2) und dem Reservoir (9) angeordneten Rückflusskanal (17) gebildet ist.

21. Magenband nach Anspruch 20, **dadurch gekennzeichnet, dass** im Rückflusskanal (17) ein Drosselventil (15) angeordnet ist.

22. Magenband nach einem der Ansprüche 2 bis 21, **dadurch gekennzeichnet, dass** die sensorische Kammer (1) mit einem Magenschrittmacher oder einem elektrische Impulse abgebenden Gerät in Verbindung steht, so dass in Abhängigkeit des in der sensorischen Kammer (1) erfassten Drucks im Magen bzw. Ösophagus eine Stimulierung der Magenwand durch elektrische Impulse über entsprechend platzierte Sonden erzielbar ist.

23. Magenband nach einem der Ansprüche 1 bis 22, **dadurch gekennzeichnet, dass** eine weitere flüssigkeitsgefüllte Kammer (18) zur Anpassung des Magenbandes vorgesehen ist.

24. Magenband nach einem der Ansprüche 23, **dadurch gekennzeichnet, dass** die weitere Kammer (18) mit einem subkutan anzuordnenden Port (20) verbunden ist, so dass durch Zuführung bzw. Abführung von Flüssigkeit über den Port (20) die Kammer (18) mit Flüssigkeit befüllt bzw. die Kammer (18) Flüssigkeit enzogen werden kann.

## Claims

1. A controllable gastric band including a nonextensible back (4) and a chamber (2) arranged on the stoma side of the back (4), for controlling the restriction of the stoma by supplying and discharging liquid to and from said chamber (2), wherein a second chamber (1) is provided, **characterized in that** the second chamber (1) is provided on the stoma side of the back (4) and communicates with the first chamber (2) in a manner to ensure the control of the stoma restriction by a displacement of the liquid between the one chamber (1) and the other chamber (2).

2. A controllable gastric band including a nonextensible back (4) and a chamber (2) arranged on the stoma side of the back (4), for controlling the restriction of the stoma by supplying and discharging liquid to and from said chamber (2), wherein a second chamber (1) is provided, **characterized in that** the second chamber (1) is provided on the stoma side of the back (4) and designed as a sensor for the detection of a pressure increase in the stomach or esophagus, and that the other chamber (2) via the sensory chamber (1) is connected with a reservoir (9) in a manner to ensure the control of the stoma restriction by a displacement of the liquid between the reservoir (9) and the stoma-restricting chamber (2) as a function of the detected pressure.

3. A gastric band according to claim 1 or 2, wherein the chambers (1, 2) are arranged one beside the other, with the stoma-restricting chamber (2) being located aborally.

4. A gastric band according to claim 3, wherein the second chamber (1) is subdivided into two communicating chambers delimiting the stoma-restricting chamber (2) on either side.

5. A gastric band according to claim 1 or 2, wherein the stoma-restricting chamber (2) and the sensory chamber (1) are arranged one above the other in respect of the stomach or esophagus, with the sensory chamber (1) being arranged on the gastric wall (13).

6. A gastric band according to claim 5, wherein a layer (16) is provided between the chambers (1, 2).

7. A gastric band according to any one of claims 1 to 6, wherein a device for pumping liquid from one chamber (1), or the reservoir (9), into the stoma-restricting chamber (2) and vice versa is provided.

8. A gastric band according to claim 7, wherein the pumping device is comprised of an electric pump (12).

9. A gastric band according to claim 7, wherein the pumping device is comprised of a mechanically driven pump, for instance a lift-and-force pump (14).

10. A gastric band according to any one of claims 1 to 9, wherein a device for detecting the eating activity is provided.

11. A gastric band according to claim 10, wherein the detection device is comprised of a device for detecting the deglutition activity.

12. A gastric band according to claim 10, wherein the detection device is comprised of a device for detecting the pressure prevailing at the gastric wall (13) or wall of the esophagus.

13. A gastric band according to claim 12, wherein a pressure sensor is provided in the sensory chamber (1) to detect the pressure prevailing at the gastric wall (13) or wall of the esophagus, said pressure sensor being connected with an electronic circuit (11).

14. A gastric band according to claim 10, wherein the detection device is comprised of a device for detecting the peristaltic wave.

15. A gastric band according to any one of claims 10 to 14, wherein the detection device is connected with the pumping device in a manner that, after having detected the eating activity, liquid is pumped from the sensory chamber (1), or reservoir (9), into the stoma-restricting chamber (2) and the liquid is again returned from the stoma-restricting chamber (2) into the second chamber (1), or reservoir (9), at a given time after the detection of a stop of the eating activity.

16. A gastric band according to claim 1, wherein the chambers (1, 2) are connected with each other via an auxiliary chamber (3), wherein a valve (5) is arranged between the second chamber (1) and the auxiliary chamber (3), which valve allows the transport of liquid only from the second chamber (1) to the auxiliary chamber (3), and wherein a further valve (6) is arranged between the auxiliary chamber (3) and the stoma-restricting chamber (2), which further valve allows the transport of liquid only from the auxiliary chamber (3) to the stoma-restricting chamber (2).

17. A gastric band according to any one of claims 2 to 15, wherein an auxiliary chamber (3) functioning as an air chamber is arranged between the reservoir (9) and the stoma-restricting chamber (2).

18. A gastric band according to any one of claims 1 to 17, wherein a device for carrying out a liquid exchange from the stoma-restricting chamber (2) to the second chamber (1) or reservoir (9), respectively, is provided between the stoma-restricting chamber (2) and the second chamber (1) or reservoir (9), respectively.

19. A gastric band according to claim 18, wherein said device for carrying out a liquid exchange is comprised of a common partition wall containing micropores and arranged between the chambers (1, 2) or between the stomach-restricting chamber (2) and the reservoir (9), respectively.

20. A gastric band according to claim 18, wherein the device for carrying out a liquid exchange is comprised of a backflow channel (17) arranged between the chambers (1, 2) or the stoma-restricting chamber (2) and the reservoir (9), respectively.

21. A gastric band according to claim 20, wherein a throttle valve (15) is arranged within the backflow channel (17).

22. A gastric band according to any one of claims 2 to 21, wherein the sensory chamber (1) is connected to a stomach pacemaker or a device emitting electric pulses so as to obtain, via appropriately placed probes, a stimulation of the gastric wall by electric pulses as a function of the pressure prevailing in the stomach or esophagus and detected by the sensory chamber (1).

23. A gastric band according to any one of claims 1 to 22, wherein a further liquid-filled chamber (18) is provided for the adaptation of the gastric band.

24. A gastric band according to claim 23, wherein said further chamber (18) is connected with a port (20) to be subcutaneously arranged in a manner that liquid can be filled into, or removed from, said chamber (18) by supplying or discharging liquid through said port (20).

## Revendications

1. Bande gastrique ajustable de manière commandée, comprenant un dos (4) non extensible et une chambre (2) agencée côté stomacal du dos (4) pour commander le rétrécissement stomacal par l'amenée ou l'évacuation de liquide dans la chambre (2) et respectivement hors de la chambre (2), une deuxième chambre (1) étant prévue en supplément, **caractérisée en ce que** la deuxième chambre (1) est prévue côté stomacal du dos (4) et est en liaison avec la première chambre (2) de manière à ce que la commande du rétrécissement stomacal soit obtenue par transfert de liquide entre ladite une chambre (1) et l'autre chambre (2).

2. Bande gastrique ajustable de manière commandée, comprenant un dos (4) non extensible et une chambre (2) agencée côté stomacal du dos (4) pour commander le rétrécissement stomacal par l'amenée ou l'évacuation de liquide dans la chambre (2) et respectivement hors de la chambre (2), une deuxième chambre (1) étant prévue en supplément, **caractérisée en ce que** la deuxième chambre (1) est prévue côté stomacal du dos (4) et est réalisée en tant que capteur pour relever une augmentation de la pression dans l'estomac ou l'oesophage, et **en ce que** l'autre chambre (2) est en liaison avec un réservoir (9) par l'intermédiaire de la chambre (1) de capteur, de manière à ce que la commande du rétrécissement stomacal soit obtenue, en fonction de la pression relevée, par transfert de liquide entre le réservoir (9) et la chambre (2) de rétrécissement stomacal.

3. Bande gastrique selon la revendication 1 ou 2, dans laquelle les chambres (1, 2) sont agencées côte à côte, la chambre (2) de rétrécissement stomacal étant placée de manière aborale.

4. Bande gastrique selon la revendication 3, dans laquelle la deuxième chambre (1) est subdivisée en deux chambres en communication réciproque, qui délimitent la chambre (2) de rétrécissement stomacal des deux côtés.

5. Bande gastrique selon la revendication 1 ou 2, dans laquelle la chambre (2) de rétrécissement stomacal et la chambre de capteur (1) sont agencées de manière superposée en se référant à l'estomac ou à l'oesophage, la chambre de capteur (1) étant agencée contre la paroi de l'estomac (13).

6. Bande gastrique selon la revendication 5, dans laquelle une couche (16) est agencée entre les chambres (1, 2).

7. Bande gastrique selon l'une des revendications 1 à 6, dans laquelle est prévu un dispositif pour le pompage du liquide de ladite une chambre (1) ou du réservoir (9) dans la chambre (2) de rétrécissement stomacal, et inversement.

8. Bande gastrique selon la revendication 7, dans laquelle le dispositif de pompage est formé par une pompe électrique (12).

9. Bande gastrique selon la revendication 7, dans laquelle le dispositif de pompage est formé par une pompe entraînée mécaniquement, par exemple une pompe aspirante et foulante (14).

10. Bande gastrique selon l'une des revendications 1 à 9, dans laquelle il est prévu un dispositif pour détecter l'activité de prise d'aliments ou de repas.

11. Bande gastrique selon la revendication 10, dans laquelle le dispositif de détection est formé par un dispositif pour détecter l'activité de déglutition.

12. Bande gastrique selon la revendication 10, dans laquelle le dispositif de détection est formé par un dispositif pour détecter la pression au niveau de la paroi de l'estomac (13) ou de la paroi de l'oesophage.

13. Bande gastrique selon la revendication 12, dans laquelle il est prévu dans la chambre de capteur (1), un capteur de pression pour relever la pression au niveau de la paroi de l'estomac (13) ou de la paroi de l'oesophage, qui est relié à un circuit électronique (11).

14. Bande gastrique selon la revendication 10, dans laquelle le dispositif de détection est formé par un dispositif pour détecter l'onde péristaltique.

15. Bande gastrique selon l'une des revendications 10 à 14, dans laquelle le dispositif de détection est relié au dispositif de pompage, de façon qu'après détection de l'activité de prise d'aliments, du liquide soit pompé de la chambre de capteur (1) ou du réservoir (9) dans la chambre (2) de rétrécissement stomacal, et qu'après un temps déterminé après la détection de la fin de l'activité de prise d'aliments, le liquide soit à nouveau ramené de la chambre (2) de rétrécissement stomacal dans la deuxième chambre (1) ou respectivement le réservoir (9).

16. Bande gastrique selon la revendication 1, dans laquelle les chambres (1, 2) sont reliées l'une à l'autre par l'intermédiaire d'une chambre auxiliaire (3), une soupape (5) agencée entre la deuxième chambre (1) et la chambre auxiliaire (3) n'autorisant un transport de liquide que de la deuxième chambre (1) vers la chambre auxiliaire (3), et une autre soupape (6) agencée entre la chambre auxiliaire (3) et la chambre (2) de rétrécissement stomacal n'autorisant un transport du liquide que de la chambre auxiliaire (3) vers la chambre (2) de rétrécissement stomacal.

17. Bande gastrique selon l'une des revendications 2 à 15, dans laquelle entre le réservoir (9) et la chambre (2) de rétrécissement stomacal est agencée une chambre auxiliaire (3) avec une fonction de réservoir d'air.

18. Bande gastrique selon l'une des revendications 1 à 17, dans laquelle il est prévu entre la chambre (2) de rétrécissement stomacal et la deuxième chambre (1) ou le réservoir (9), un dispositif pour effectuer un échange de liquide de la chambre (2) de rétrécissement stomacal vers la deuxième chambre (1) ou respectivement vers le réservoir (9).

19. Bande gastrique selon la revendication 18, dans laquelle le dispositif pour effectuer un échange de liquide est formé par une paroi de séparation commune entre les chambres (1, 2) ou respectivement entre la chambre (2) de rétrécissement stomacal et le réservoir (9), qui renferme des micropores.

20. Bande gastrique selon la revendication 18, dans laquelle le dispositif pour effectuer un échange de liquide est formé par un canal d'écoulement de retour (17) agencé entre les chambres (1, 2) ou respectivement entre la chambre (2) de rétrécissement stomacal et le réservoir (9).

21. Bande gastrique selon la revendication 20, dans laquelle une soupape d'étranglement (15) est agencée dans le canal d'écoulement de retour (17).

22. Bande gastrique selon l'une des revendications 2 à 21, dans laquelle la chambre de capteur (1) est en liaison avec un stimulateur gastrique ou un appareil délivrant des impulsions électriques, de manière à obtenir, en fonction de la pression dans l'estomac ou l'oesophage relevée dans la chambre de capteur (1), une stimulation de la paroi de l'estomac par des impulsions électriques par l'intermédiaire de sondes placées de manière appropriée.

23. Bande gastrique selon l'une des revendications 1 à 22, dans laquelle il est prévu une autre chambre (18) remplie de liquide, pour l'ajustement ou l'adaptation de la bande gastrique.

24. Bande gastrique selon la revendication 23, dans laquelle ladite autre chambre (18) est reliée à un port de branchement (20) à implanter en mode sous-cutané, de manière à ce que par amenée ou évacuation de liquide par l'intermédiaire du port de branchement (20), la chambre (18) puisse être remplie avec du liquide ou bien respectivement qu'il soit possible de soutirer du liquide de la chambre (18).
